# EUROPÄISCHE PATENTSCHRIFT

(11) **EP 3 434 378 B1**
(45) Veröffentlichungstag und Bekanntmachung des Hinweises auf die Patenterteilung: **04.09.2024**
(21) Anmeldenummer: 18182651.2
(22) Anmeldetag: 10.07.2018
(51) Int. Cl.: B05D 3/14, B05D 3/02, C09J 5/02

(54) **VERFAHREN ZUR BESCHICHTUNG UND WERKSTÜCK**
WORKPIECE AND METHOD FOR COATING
PROCÉDÉ DE REVÊTEMENT ET PIÈCE À USINER

(30) Priorität: 27.07.2017 DE 102017212974
(43) Veröffentlichungstag der Anmeldung: 30.01.2019
(73) Patentinhaber: Gerresheimer Bünde GmbH, 32257 Bünde (DE)
(72) Erfinder: Josten, Stefan, 33613 Bielefeld (DE); Wittland, Frank, 32130 Enger (DE); Brückner, Stephan, 37318 Mackenrode (DE); Gerhard, Christoph, 37133 Friedland (DE); Viöl, Wolfgang, 37139 Adelebsen (DE)
(74) Vertreter: Friese Goeden Patentanwälte PartGmbB

(56) Entgegenhaltungen:
- EP-A1- 0 709 105
- EP-A1- 1 352 667
- WO-A1-2016/124824
- WO-A1-99/57185
- DE-A1- 102008 009 171
- FR-A1- 2 840 826
- JP-A- H0 341 176
- US-A- 4 524 089
- US-A1- 2010 304 156

## Beschreibung

Die Erfindung betrifft ein Verfahren zur Beschichtung, bei welchem zumindest auf eine erste Teilfläche einer Oberfläche eines Bauteils zumindest eine Emulsion und/oder eine Lösung aufgebracht wird, welche zumindest eine schichtbildende Substanz enthält, und nachfolgend das Bauteil wärmebehandelt wird. Weiterhin betrifft die Erfindung ein Verfahren, bei welchem zumindest ein solches Bauteil mit zumindest einem weiteren Bauteil gefügt wird. Schließlich betrifft die Erfindung ein solchermaßen erhaltenes Werkstück.

Ein gattungsgemäßes Verfahren ist beispielsweise aus der EP 1 352 667 A1 bekannt. Dieses Dokument beschreibt ein Verfahren zum Herstellen einer Injektionsspritze, bei welchem der Spritzenkörper bei hoher Temperatur innen mit einem Gleitmittel versehen wird. An der Fügestelle zwischen Spritzenkörper und Kanüle wird das Gleitmittel darauffolgend wieder entfernt, um eine zuverlässige Haftung des zum Fügen verwendeten Klebstoffes zu ermöglichen.

Dieses bekannte Verfahren weist den Nachteil auf, dass das vollständige Entfernen des Gleitmittels in einem räumlich eng begrenzten Bereich nur mit großem Aufwand erreicht werden kann. Ausgehend von diesem bekannten Verfahren liegt der Erfindung somit die Aufgabe zugrunde, zumindest eine Teilfläche einer Beschichtung in einfacher Weise für eine nachfolgende Klebeverbindung oder auch ein weiteres Beschichtungsverfahren vorzubereiten.

Aus der WO 2016/124824 A1 ist ein Verfahren zum Einkapseln einer Verglasung in Polycarbonat bekannt. Das Verfahren umfasst die folgenden aufeinanderfolgenden Schritte:
(a) Behandeln eines Bereichs der Oberfläche der Verglasung, der eine harte Beschichtung auf Silikonbasis trägt, mit atmosphärischem Plasma unter Verwendung einer Plasmadüse
(b) Auftragen einer primären Zusammensetzung, umfassend einen oder mehrere Haftvermittler, ausgewählt aus Diisocyanaten, Polyisocyanaten und chloriertem Polyolefin,
(c) Verdampfen des Lösungsmittels und
(d) Überformen eines thermoplastischen Polymers auf dem Bereich, der von der trockenen Grundierungsschicht bedeckt ist.

Die WO 99/57185 A1 offenbart einen Artikel mit einer gemusterten Oberfläche, welche abwechselnd hydrophobe und hydrophile Oberflächenbereiche enthält. Die hydrophoben Bereiche können ausreichend schmal sein, so dass unter Taubedingungen Feuchtigkeit, die sich auf dem hydrophoben Bereich angesammelt hat, zu dem hydrophilen Bereich wandert, wodurch die Ansammlung von Wassertröpfchen verhindert wird. Bei Frostbedingungen bleibt der hydrophobe Bereich relativ frostfrei, wodurch eine zumindest teilweise Transparenz der Oberfläche aufrechterhalten wird.

US 4,524,089 A1 zeigt eine dreistufige Plasmabehandlung zur Verbesserung der Laminathaftung von metallischen und nichtmetallischen Substraten. Die Behandlung umfasst das aufeinanderfolgende Aussetzen des Substrats einem ersten Plasma aus Sauerstoffgas, einem zweiten Plasma aus einem Kohlenwasserstoffmonomergas und einem dritten Plasma aus Sauerstoffgas.

Aus der JP H03 41176 A ist ein Verfahren bekannt welches die verbesserte Luftdichtigkeit einer Fügestelle ermöglicht. Hierzu erfolgt eine spezielle Behandlung der Oberfläche einer inneren Harzschicht eines Schlauchs an dem Teil, an dem ein Mundstück angebracht werden soll. Nachfolgend wird ein aushärtbarer Klebstoff auf die Außenseite des Mundstücks aufgetragen und das Mundstück in den Schlauch eingesetzt.

Die FR 2 840 826 A1 zeigt ein Verfahren zur Oberflächenbehandlung eines Artikels, der vernetztes Silikon umfasst, durch einen Plasmastrahl unter Verwendung eines homogenen atmosphärischen Plasmas, das durch eine Plasmaprojektionseinrichtung erzeugt wird. Die Plasmaprojektionseinrichtung umfasst einen rotierenden Kopf, der eine oder mehrere Plasmadüsen aufweist.

US 2010/304156 A1 offenbart ein Fügeverfahren mit folgenden Schritten: Vorbereiten eines ersten Grundmaterials und eines zweiten Grundmaterials, die über einen Verbindungsfilm miteinander verbunden werden sollen; Bilden einer flüssigen Beschichtung durch Auftragen eines flüssigen Materials, das ein Polyester-modifiziertes Silikonmaterial enthält, auf mindestens eines der Grundmaterialien; Trocknen und/oder Härten der flüssigen Beschichtung, um den Verbindungsfilm zu erhalten; Behandeln des Verbindungsfilms mit einem Plasma und Inkontaktbringen des ersten Grundmaterials und des zweiten Grundmaterials über den Verbindungsfilm.

Die Aufgabe wird erfindungsgemäß durch ein Verfahren gemäß Anspruch 1 und ein Werkstück nach Anspruch 10 gelöst. Vorteilhafte Weiterbildungen der Erfindung finden sich in den Unteransprüchen.

Erfindungsgemäß wird ein Verfahren zur Beschichtung vorgeschlagen, bei welchem zumindest auf eine Teilfläche einer Oberfläche eines Bauteils zumindest eine Emulsion und/oder eine Lösung aufgebracht wird, welche zumindest eine schichtbildende Substanz enthält. Das Bauteil kann in einigen Ausführungsformen der Erfindung aus Glas, Kunststoff, einem Metall oder einer Legierung gefertigt sein. Das Bauteil weist eine gekrümmte Oberfläche auf.

Insbesondere ist das Bauteil eine Hohlform, deren Innenfläche mit einer Beschichtung versehen wird. Das Bauteil kann in einigen Ausführungsformen der Erfindung ein Halbzeug sein, welches in nachfolgenden Verfahrensschritten zu einem Fertigprodukt weiterbearbeitet wird. In anderen Ausführungsformen der Erfindung kann das Bauteil auch ein Fertigprodukt sein, welches nach Durchführung des vorgeschlagenen Beschichtungsverfahrens keiner weiteren Bearbeitung mehr bedarf.

Die Beschichtung enthält zumindest eine schichtbildende Substanz, welche in Form einer Emulsion und/oder einer Lösung vorliegt. Die Emulsion oder die Lösung enthält neben der schichtbildenden Substanz zumindest ein Lösemittel. Für die Zwecke der vorliegenden Beschreibung wird die Trägermatrix einer Emulsion ebenfalls als ,Lösemittel' bezeichnet, auch wenn die Schichtbildenden Substanzen nur emulgiert und nicht im strengeren Sinne gelöst sind.

Die Emulsion oder die Lösung kann durch an sich bekannte Verfahren auf zumindest eine erste Teilfläche des Bauteils aufgebracht werden. In einigen Ausführungsformen der Erfindung kann auch eine Vollbeschichtung des Bauteils vorgesehen sein, d.h. die gesamte Oberfläche des Bauteils wird erfindungsgemäß beschichtet. Die Emulsion oder die Lösung kann beispielsweise durch Sprühen, Drucken, Streichen oder Tauchen aufgebracht werden. Um eine gleichmäßigere Beschichtung mit der Emulsion bzw. der Lösung zu ermöglichen, können zusätzliche Maßnahmen ergriffen werden, beispielsweise das Herabsetzen der Viskosität durch Erwärmen und/oder Verdünnen oder eine Unterstützung der Beschichtung durch elektrische Felder ähnlich einer Elektrotauchlackierung.

Erfindungsgemäß wird das Bauteil nach dem Aufbringen der Emulsion bzw. der Lösung wärmebehandelt. Die Wärmebehandlung kann unmittelbar nach dem Aufbringen der Emulsion bzw. der Lösung erfolgen. In anderen Ausführungsformen der Erfindung kann die Wärmebehandlung nach einem vorangegangenen Trocknungsschritt durchgeführt werden. Die Wärmebehandlung führt einerseits dazu, dass das Lösungsmittel der Emulsion bzw. der Lösung verdampft bzw. aus der Schicht ausgetrieben wird. Darüber hinaus kann eine Verbindung zwischen den schichtbildenden Substanzen und der Oberfläche des Bauteils entstehen, beispielsweise durch kovalente Bindungen oder Van-der-Waals-Bindungen. In einigen Ausführungsformen der Erfindung können die schichtbildenden Substanzen auch untereinander vernetzt sein und beispielsweise ein Polymer oder eine polymerartige Verbindung ausbilden.

In einigen Ausführungsformen der Erfindung ist die auf diese Weise erzeugte Beschichtung hydrophob und/oder weist gegenüber der unbehandelten Oberfläche verbesserte Gleiteigenschaften auf. In einigen Ausführungsformen der Erfindung kann die erzeugte Beschichtung auch chemisch inert gegenüber Gasen oder Flüssigkeiten sein, mit welchen das Bauteil bei bestimmungsgemäßem Gebrauch in Kontakt kommt.

In einem nachfolgenden Verfahrensschritt wird zumindest eine zweite Teilfläche der mit der Beschichtung versehenen ersten Teilfläche des Bauteils einer Plasmabehandlung unterzogen, um auf diese Weise die Wirkungen der Beschichtung zumindest teilweise zu deaktivieren bzw. rückgängig zu machen, ohne die Beschichtung als solche vollständig zu entfernen. Unter einem Plasma im Sinne der vorliegenden Beschreibung wird ein teilweise ionisiertes Gas verstanden, welches bei einem vorgebbaren Druck und mit vorgebbarer Zusammensetzung für eine bestimmte Zeitdauer auf zumindest eine zweite Teilfläche der Beschichtung einwirkt. In einigen Ausführungsformen der Erfindung kann die zweite Teilfläche ebenso groß sein wie die beschichtete erste Teilfläche, sodass das Plasma auf die gesamte vorher aufgebrachte Beschichtung einwirkt. Sofern die erste Teilfläche die gesamte Oberfläche des Bauteils umfasst, kann das Plasma auch auf die gesamte Oberfläche des Bauteils einwirken. In anderen Ausführungsformen der Erfindung wird nur ein Teil der Beschichtung der Plasmabehandlung unterworfen, wohingegen andere Teile der Beschichtung nicht dem Plasma ausgesetzt werden. Beispielsweise können Teilflächen der Plasmabehandlung unterzogen werden, welche in einem weiteren Verfahrensschritt eine Fügestelle ausbilden sollen, um auf diese Weise die Haftung von Klebstoffen oder Lot zu verbessern oder überhaupt erst zu ermöglichen.

Durch die Einwirkung des Plasmas reduziert sich der Kohlenstoffgehalt der Beschichtung auf weniger als etwa 80% oder weniger als etwa 75% oder weniger als etwa 70% oder weniger als etwa 60% des Ausgangswertes vor der Plasmabehandlung. Die Reduktion des Kohlenstoffgehaltes kann darüber hinaus auch die Wirkung haben, dass sich die Bindungen innerhalb der Beschichtung ändern. Beispielsweise kann die Beschichtung von einer polymeren Struktur in eine glasartige oder amorphe Struktur umgewandelt werden.

Schließlich kann durch die Einwirkung des Plasmas die Schichtdicke in der behandelten zweiten Teilfläche abnehmen. In einigen Ausführungsformen der Erfindung kann die Schichtdicke um mehr als etwa 20% oder mehr als etwa 25 % oder mehr als etwa 30 % reduziert werden.

Erfindungsgemäß wurde jedoch erkannt, dass die Beschichtung durch die Einwirkung des Plasmas nicht vollständig entfernt wird. Vielmehr kann nach der Plasmabehandlung in der zweiten Teilfläche eine Schichtdicke von mehr als etwa 70% oder mehr als etwa 60 % oder mehr als etwa 50 % der im ersten Verfahrensschritt aufgebrachten Schichtdicke verbleiben.

Zusammenfassend kann festgehalten werden, dass durch die Plasmabehandlung die Bindungsverhältnisse und/oder die Schichtdicke und/oder das Elementinventar der ursprünglich aufgebrachten Beschichtung in der dem Plasma ausgesetzten Teilfläche verändert werden. Hierdurch kann eine Aufrauung der Beschichtung und/oder eine Hydrophilierung eintreten, sodass ein nachfolgendes Beschichtungs- oder Fügeverfahren erleichtert oder überhaupt erst ermöglicht wird. Gleichzeitig kann die Plasmabehandlung einfacher durchzuführen sein als eine vollständige Entfernung der Beschichtung, welche unter Umständen sehr stark an der Oberfläche des Bauteils haftet und nur mechanisch oder nasschemisch entfernt werden kann, wobei einerseits die entstehende Umweltbelastung erfindungsgemäß vermieden werden kann und andererseits aufwendige Maßnahmen zur Beschränkung des Zutritts eines flüssigen oder gasförmigen Ätzmediums außerhalb der zu behandelnden zweiten Teilflächen vermieden werden kann.

In einigen Ausführungsformen der Erfindung beträgt die Schichtdicke der Beschichtung zumindest in der zweiten Teilfläche vor der Einwirkung des Plasmas zwischen etwa 20 nm und etwa 100 nm oder zwischen etwa 30 nm und etwa 70 nm. Eine solche Beschichtung beeinträchtigt die Maßhaltigkeit des beschichteten Bauteils nicht, kann jedoch bereits ausreichen, um verbesserte Gleiteigenschaften, eine Hydrophobierung und/oder eine Passivierung der Oberfläche zu bewirken.

In einigen Ausführungsformen der Erfindung kann die Emulsion und/oder die Lösung zumindest ein Silikonöl und ein Lösungsmittel enthalten oder daraus bestehen. In einigen Ausführungsformen der Erfindung kann das Lösungsmittel Wasser sein oder Wasser enthalten. In anderen Ausführungsformen der Erfindung kann das Lösungsmittel aliphatische Kohlenwasserstoffe oder Aromaten enthalten, beispielsweise Hexan, Heptan, Toluol und/oder Xylol. Schließlich kann das Lösungsmittel zur Bildung einer Emulsion auch einen Alkohol und/oder Glyzerin und/oder Äther enthalten. In jedem Fall kann das Lösungsmittel entweder bei normalen Umgebungsbedingungen oder bei erhöhter Temperatur im Wärmeschrank oder Ofen verdampft bzw. aus der entstehenden Beschichtung ausgetrieben werden, sodass eine Beschichtung auf der ersten Teilfläche des Bauteils verbleibt, welche Silikon enthält oder daraus besteht.

Die Beschichtung kann in einigen Ausführungsformen der Erfindung zumindest Kohlenstoff und Sauerstoff und Wasserstoff und Silizium enthalten. In einigen Ausführungsformen der Erfindung kann die Beschichtung zumindest ein Polyorganosiloxan enthalten oder daraus bestehen. Durch die Einwirkung des Plasmas kann der Kohlenstoff aus der Beschichtung zumindest teilweise entfernt werden, sodass die Beschichtung nach der Plasmabehandlung Siliziumoxid und/oder Siliziumnitrid und/oder Siliziumoxinitrid enthält oder daraus besteht.

In einigen Ausführungsformen der Erfindung ist die zweite Teilfläche vor der Plasmabehandlung hydrophob und nach der Plasmabehandlung hydrophil. In anderen Ausführungsformen der Erfindung ist die zweite Teilfläche vor der Plasmabehandlung hydrophil und nach der Plasmabehandlung hydrophob. Unter einer hydrophoben Beschichtung wird für die Zwecke der vorliegenden Beschreibung eine Oberfläche verstanden, welche bei Kontakt mit Wasser einen Kontaktwinkel von mehr als 90° ausbildet. Eine hydrophile Oberfläche ist für die Zwecke der vorliegenden Beschreibung eine Oberfläche, welche bei Benetzung mit Wasser einen Kontaktwinkel von weniger als 90° ausbildet. Durch die Plasmabehandlung kann die Oberflächenenergie daher soweit geändert werden, dass die Haftfestigkeit bzw. die Benetzbarkeit mit einem Schmiermittel, einem Klebstoff, einem Lot oder einem Lack ansteigt bzw. überhaupt erst ermöglicht wird.

In einigen Ausführungsformen der Erfindung kann das Plasma (4) für etwa 0,4 bis etwa 5 Sekunden oder für etwa 0,5 bis etwa 4 Sekunden oder für etwa 0,5 bis etwa 1,5 Sekunden oder für etwa 5 bis etwa 60 Sekunden einwirken. Erfindungsgemäß wurde erkannt, dass diese kurze Behandlungsdauer bereits ausreicht, um eine hydrophobe Silikonbeschichtung in eine hydrophile, siliziumhaltige Verbindung umzusetzen, sodass das erfindungsgemäß vorgeschlagene Verfahren auch bei der Herstellung von Massenprodukten wirtschaftlich eingesetzt werden kann.

In einigen Ausführungsformen der Erfindung kann ein Atmosphärendruckplasma zum Einsatz kommen, welches mit einer dielektrisch behinderten Entladung erzeugt wird. Dieses Merkmal hat die Wirkung, dass auf eine aufwendige Vakuumtechnik verzichtet werden kann und das Verfahren auf diese Weise leicht in bestehende Produktionsprozesse integriert werden kann. Die Verwendung einer dielektrisch behinderten Entladung sorgt dabei dafür, dass die in das Plasma eingekoppelte elektrische Leistung begrenzt bleibt und thermische Schäden des Bauteils und/oder der Beschichtung vermieden werden können.

Erfindungsgemäß kommt zur Erzeugung des Plasmas eine Wirbelströmung des Arbeitsgases zum Einsatz. Dadurch kann die Entladung in die Länge gezogen werden. Insbesondere die Behandlung der Innenflächen zylindrischer Hohlkörper verstärkt diesen Effekt noch, da die Oberflächen die Entladung unterstützen. Dadurch können auch schlanke Gegenstände wie Rohre oder Spritzen behandelt werden.

In einigen Ausführungsformen der Erfindung kann das Plasma ein Inertgas enthalten oder daraus bestehen. In einigen Ausführungsformen der Erfindung kann das Inertgas ein Edelgas sein oder ein solches enthalten. In einigen Ausführungsformen der Erfindung kann das Plasma Argon enthalten oder daraus bestehen. Ein Inertgasplasma sorgt insbesondere dafür, dass die Bestandteile der Beschichtung nicht oxidiert oder reduziert werden oder anderweitig mit dem Prozessgas des Plasmas reagieren, so dass sich die Beschichtung nicht in unerwünschter Weise umsetzt oder durch reaktives Ätzen von der Oberfläche entfernt wird.

In einigen Ausführungsformen der Erfindung kann das Plasma ein Aktivgas sein, welches beispielsweise Stickstoff und/oder Sauerstoff enthält oder daraus besteht.

In einigen Ausführungsformen der Erfindung kann das Plasma durch eine Wechselspannung oder eine gepulste Spannung erzeugt werden, welche eine Frequenz zwischen etwa 10 kHz und etwa 30 kHz oder zwischen etwa 15 kHz und etwa 25 kHz aufweist.

In einigen Ausführungsformen der Erfindung kann ein Plasmastrahl verwendet werden, welcher durch ein elektrisches Feld zwischen der Oberfläche des Bauteils und zumindest einer Gegenelektrode durch Ionisieren eines Arbeitsgasstromes erzeugt wird. In anderen Ausführungsformen der Erfindung kann ein Plasmajet verwendet werden. In diesem Fall wird das Plasma im Inneren der Plasmaquelle durch elektrische Felder oder elektromagnetische Strahlung erzeugt und vom Arbeitsgasstrom aus der Quelle ausgetrieben. In einigen Ausführungsformen der Erfindung wird das Plasma mit einer dielektrisch behinderten Entladung erzeugt. Solchermaßen erzeugte Plasmen weisen nur geringe Temperaturerhöhungen von weniger als etwa 50 K oder weniger als etwa 30 K gegenüber der Umgebung auf, sodass thermische Schäden auch bei empfindlichen Oberflächen vermieden werden können. Dies ist beispielsweise bei der Behandlung von Bauteilen aus einem Polymer oder anderen Kunststoffen hilfreich.

In einigen Ausführungsformen der Erfindung betrifft diese ein Verfahren zur Herstellung eines Werkstückes mit zumindest einem ersten Bauteil und zumindest einem zweiten Bauteil, welche miteinander gefügt werden. Dabei wird zumindest ein Bauteil zunächst wie vorstehend beschrieben beschichtet und sodann zumindest die zur Ausbildung der Fügestelle vorgesehene zweite Teilfläche mit dem erfindungsgemäßen Plasma behandelt, um die Beschichtung in der Teilfläche wie beschrieben zu verändern. Auf diese Weise wird die Haftung eines Lotes oder eines Klebstoffes verbessert, sodass die Fügestelle eine verbesserte Dichtigkeit und/oder größere Haftfestigkeiten aufweist.

In einigen Ausführungsformen der Erfindung kann das Fügen unter Verwendung eines Klebstoffes erfolgen, welcher insbesondere ausgewählt ist aus einem Acrylat und/oder Polyurethan und/oder einem Epoxidharz und/oder einem Cyanacrylat. Solche Klebstoffe weisen hohe Haftfestigkeiten bei einfacher Verarbeitbarkeit und hohen Anfangsfestigkeiten auf, sodass die Produktion des Werkstückes rasch vorgenommen werden kann.

In einigen Ausführungsformen der Erfindung betrifft diese ein Werkstück, welches zumindest ein erstes Bauteil und zumindest ein zweites Bauteil umfasst, welche durch Kleben miteinander gefügt sind, wobei die Fügestelle zumindest eine zweite Teilfläche einer ersten Teilfläche der Oberfläche des ersten Bauteils umfasst, wobei zumindest auf der ersten Teilfläche der Oberfläche des ersten Bauteils zumindest eine Beschichtung aufgebracht ist. Zumindest die zweite Teilfläche der beschichteten ersten Teilfläche wurde nachfolgend einem Plasma ausgesetzt, wodurch der Kohlenstoffgehalt der Beschichtung reduziert und/oder die Bindungsverhältnisse der Beschichtung geändert und/oder die Schichtdicke reduziert wurde. Die beschriebenen Veränderungen können eine Erhöhung der Haftfestigkeit der Klebeverbindung bewirken, sodass die Zuverlässigkeit des Werkstückes erhöht sein kann. Das Werkstück kann ein fertiges Produkt oder ein Halbzeug sein, welches in nachfolgenden Verfahrensschritten weiter bearbeitet wird.

Erfindungsgemäß ist der Kohlenstoffgehalt der Beschichtung in der zweiten Teilfläche der Beschichtung (3) auf weniger als etwa 80% oder weniger als etwa 75% oder weniger als etwa 70% oder weniger als etwa 60% des Ausgangswertes vor der Plasmabehandlung reduziert.

Das Werkstück kann in einigen Ausführungsformen der Erfindung ausgewählt sein aus einer Fertigspritze und/oder einer Verpackung und/oder einer Maschinenkomponente. Eine Maschinenkomponente kann beispielsweise ein Gehäuse sein, welches andere Komponenten umgibt. Zumindest das erste und/oder das zweite Bauteil des Werkstücks kann aus einem Kunststoff, einem Glas, einem Metall oder einer Legierung gefertigt sein.

Nachfolgend soll die Erfindung anhand von Figuren ohne Beschränkung des allgemeinen Erfindungsgedankens näher erläutert werden. Dabei zeigt
- Figur 1: ein zur Beschichtung vorgesehenes Bauteil.
- Figur 2: zeigt ein Bauteil mit aufgebrachter Beschichtung.
- Figur 3: erläutert die Plasmabehandlung einer Teilfläche des Bauteils.
- Figur 4: zeigt das Bauteil nach erfolgter Plasmabehandlung.
- Figur 5: zeigt eine Plasmaquelle, welche mit dem vorgeschlagenen Verfahren verwendbar ist.
- Figur 6: zeigt in einem Ausführungsbeispiel die Schichtdickenabnahme über die Behandlungszeit.
- Figur 7: zeigt Messergebnisse der Photoelektronenspektroskopie an einer erfindungsgemäßen Beschichtung.
- Figur 8: zeigt ein Werkstück gemäß einer ersten Ausführungsform der Erfindung.
- Figur 9: zeigt ein Werkstück gemäß einer zweiten Ausführungsform der Erfindung.
- Figur 10: zeigt ein Werkstück gemäß einer dritten Ausführungsform der Erfindung.
- Figur 11: zeigt die Abnahme des Kohlenstoffgehaltes gegen die Behandlungsdauer.
- Figur 12: zeigt ein Werkstück gemäß einer vierten Ausführungsform der Erfindung.

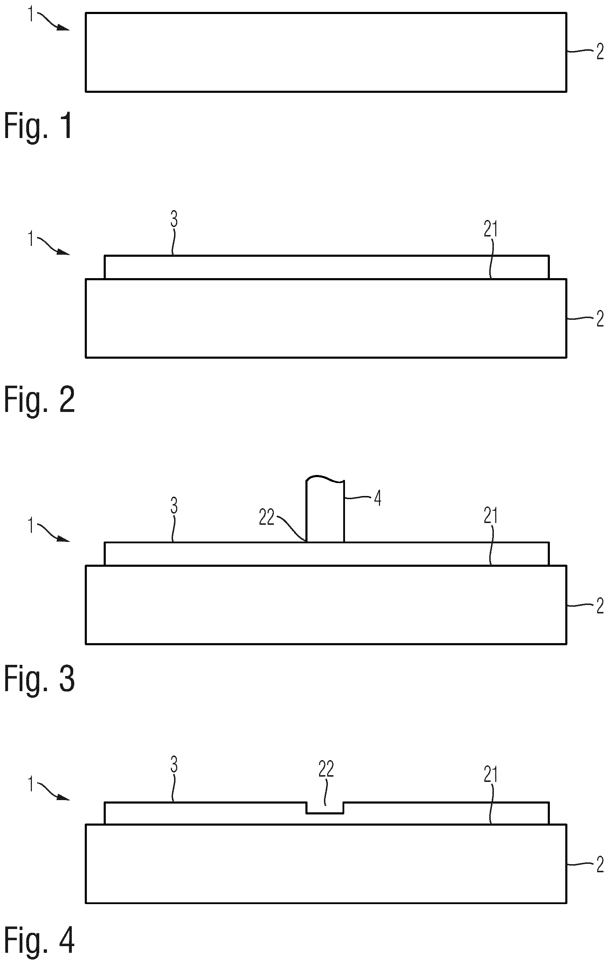

Figur 1 zeigt ein Bauteil 2 als Teil eines Werkstückes. Das Bauteil 2 ist in Figur 1 schematisch als planparallele Platte dargestellt. In anderen Ausführungsformen der Erfindung kann das Bauteil 2 selbstverständlich auch komplexere Formen annehmen, beispielsweise die Form eines Hohlzylinders oder eines komplexen Maschinenelementes.

Das Bauteil 2 kann beispielsweise aus Glas, Kunststoff, einem Metall oder einer Legierung gefertigt sein. Das Bauteil 2 kann in einigen Ausführungsformen der Erfindung ein Kompositmaterial sein bzw. daraus bestehen, welches aus verschiedenen Materialien zusammengesetzt ist. Die vereinfachte, rein illustrative Darstellung des Bauteils 2 in Figur 1 soll den Gegenstand der Erfindung nicht beschränken.

Figur 2 zeigt das Aufbringen einer Beschichtung 3 in einer ersten Teilfläche 21 des Bauteils 2. In einigen Ausführungsformen der Erfindung kann die erste Teilfläche 21 die gesamte Oberfläche des Bauteils 2 umfassen oder die gesamte Fläche einer Kavität oder eines Innenraumes. In anderen Ausführungsformen der Erfindung kann die erste Teilfläche 21 eine Teilbeschichtung der Oberfläche des Bauteils 2 darstellen, so dass andere Teilflächen unbeschichtet bleiben oder mit anderem Beschichtungsmaterial oder anderen Beschichtungsverfahren beschichtet werden.

Die Beschichtung 3 wird durch Aufbringen schichtbildender Substanzen erzeugt, welche als Emulsion oder Lösung in einem Lösungsmittel vorliegen. Für die Zwecke der vorliegenden Beschreibung soll auch die Trägersubstanz einer Emulsion als Lösungsmittel bezeichnet werden, auch wenn sich die schichtbildenden Substanzen darin nicht chemisch lösen, sondern nur emulgieren bzw. dispergieren.

Als Lösungsmittel können nichtpolare Lösungsmittel, beispielsweise aromatische Kohlenwasserstoffe, Verwendung finden oder auch Wasser. Die schichtbildenden Substanzen der Beschichtung 3 können in einigen Ausführungsformen der Erfindung Siloxane oder ein Silikonöl sein.

Nach Aufbringen der Emulsion bzw. der Lösung durch Aufrakeln, Tauchen, Sprühen, Streichen oder Drucken wird das Lösungsmittel beispielsweise durch eine thermische Nachbehandlung aus der Beschichtung 3 entfernt. Durch die thermische Nachbehandlung können die schichtbildenden Substanzen darüber hinaus miteinander vernetzen und beispielsweise ein Poly(organo)siloxan bilden. Darüber hinaus können durch die Wärmebehandlung Bindungskräfte zwischen der Beschichtung 3 und dem Werkstück 2 ausgebildet werden, beispielsweise in Form kovalenter Bindungen oder Van-der-Waals-Bindungen. Dem Aufbringen der Beschichtung 3 kann ein optionaler Reinigungsschritt zumindest der ersten Teilfläche 21 vorausgehen. In anderen Ausführungsformen der Erfindung kann die Beschichtung 3 aus einer Mehrzahl von Einzelschichten bestehen, welche übereinander aufgebracht werden.

Figur 3 zeigt einen nachfolgenden Verfahrensschritt. In diesem Verfahrensschritt wird die Beschichtung 3 bzw. zumindest eine zweite Teilfläche 22 der Beschichtung 3 einem Plasma 4 ausgesetzt. Hierdurch wird die Schichtdicke und/oder die Bindungsverhältnisse und/oder das Elementinventar und/oder das Benetzungsverhalten der Beschichtung 3 in der zweiten Teilfläche 22 verändert.

Das Plasma 4 kann in einigen Ausführungsformen der Erfindung ein Atmosphärendruckplasma sein, sodass die Verwendung aufwendiger Vakuumtechnik vermieden wird. In einigen Ausführungsformen der Erfindung kann das Plasma 4 mit einer dielektrisch behinderten Entladung erzeugt werden, welche das Einbringen großer thermischer Lasten in die zweite Teilfläche 22 vermeiden kann. Das Plasma 4 kann in einigen Ausführungsformen der Erfindung ein Inertgas enthalten bzw. daraus bestehen, beispielsweise ein Edelgas wie Argon, Helium oder Xenon.

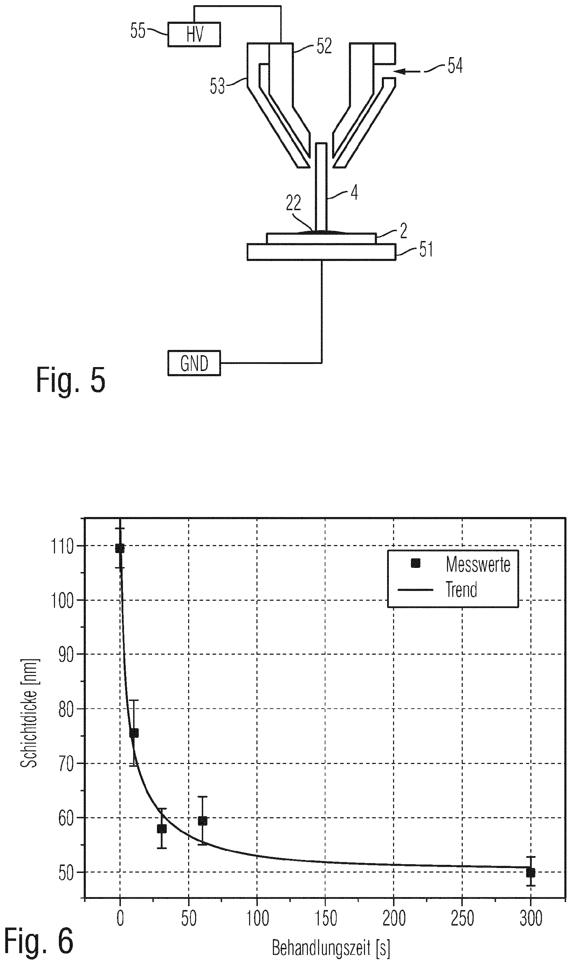

Die Einwirkdauer des Plasmas 4 kann in einigen Ausführungsformen der Erfindung zwischen etwa 45 Sekunden und etwa 120 Sekunden betragen. In anderen Ausführungsformen der Erfindung kann das Plasma für etwa 0,4 bis etwa 5 Sekunden oder für etwa 0,5 bis etwa 4 Sekunden oder für etwa 0,5 bis etwa 1,5 Sekunden oder für etwa 5 bis etwa 60 Sekunden einwirken. Erfindungsgemäß wurde erkannt, dass auch diese kurze Behandlungsdauer bereits ausreicht, um eine hydrophobe Silikonbeschichtung in eine hydrophile, siliziumhaltige Verbindung umzusetzen, sodass das erfindungsgemäß vorgeschlagene Verfahren auch bei der Herstellung von Massenprodukten wirtschaftlich eingesetzt werden kann. Ein Ausführungsbeispiel einer Plasmaquelle, mit welcher der Plasmastrahl des Plasmas 4 erzeugbar ist, wird nachfolgend anhand von Figur 5 näher erläutert.

Figur 4 zeigt die Auswirkungen der Plasmabehandlung in der zweiten Teilfläche 22 der Beschichtung 3 bzw. der Teilfläche 22 der ersten Teilfläche 21. Wie in Figur 4 dargestellt ist, führt die Plasmabehandlung zu einer Reduktion der Schichtdicke um mehr als etwa 20% oder mehr als etwa 25 % oder mehr als etwa 30 %. Es ist jedoch darauf hinzuweisen, dass die Beschichtung 3 nicht vollständig entfernt wird, sondern nach der Plasmabehandlung in der zweiten Teilfläche 22 eine Schichtdicke von mehr als etwa 70% oder mehr als etwa 60 % oder mehr als etwa 50 % verbleibt.

Darüber hinaus kann die Einwirkung des Plasmas 4 dazu führen, dass sich das Elementinventar der Beschichtung 3 ändert. Beispielsweise kann der Kohlenstoffgehalt der Beschichtung 3 auf weniger als etwa 80% oder weniger als etwa 75% oder weniger als etwa 70% oder weniger als etwa 60% des Ausgangswertes vor der Plasmabehandlung abnehmen.

Schließlich kann sich in einigen Ausführungsformen der Erfindung das Benetzungsverhalten der zweiten Teilfläche 22 ändern, d. h. die Beschichtung 3 kann ursprünglich hydrophob sein und nach Einwirkung des Plasmas 4 in der zweiten Teilfläche 22 hydrophil werden.

Anhand von Figur 5 wird eine Plasmaquelle näher erläutert, welche zur Durchführung des vorstehend beschriebenen Beschichtungsverfahrens geeignet ist. Die Plasmaquelle umfasst eine Hochspannungselektrode 52, welche von einem Isolator 53 umgeben ist. Die Hochspannungselektrode 52 ist als Hohlkörper ausgebildet und kann beispielsweise zylindrisch oder konisch sein. Der Isolierkörper 53 ist von der Hochspannungselektrode 52 beabstandet angeordnet. Auf diese Weise kann ein Arbeitsgas in den Zwischenraum zwischen der Hochspannungselektrode 52 und dem Isolierkörper 53 eingeführt werden, welches über eine Gaszufuhr 54 zugeführt werden kann. Das Arbeitsgas verlässt die Hochspannungselektrode 52 über deren substratseitige Mündung.

Der Mündung gegenüberliegend ist eine Gegenelektrode 51 angeordnet, welche optional mit einer dielektrischen Beschichtung versehen sein kann. Hierdurch wird sichergestellt, dass zwischen der Hochspannungselektrode 52 und der Gegenelektrode 51 in jedem Fall eine dielektrisch behinderte Entladung gezündet wird. Sofern das Substrat selbst ein Dielektrikum bzw. einen Isolator enthält oder daraus besteht, kann die dielektrische Beschichtung der Gegenelektrode 51 auch entfallen.

Bei Betrieb der Vorrichtung wird über die Gaszufuhr 54 ein Arbeitsgas, beispielsweise Argon, zugeführt. An die Hochspannungselektrode 52 wird eine hochfrequente Wechselspannung angelegt, welche mit einer Hochspannungsquelle 55 erzeugt wird. In einigen Ausführungsformen der Erfindung kann die Amplitude der angelegten Hochspannung zwischen etwa 2 kV bis etwa 10 kV oder zwischen etwa 5 kV und etwa 8 kV betragen. Die Hochspannung kann als sinusförmige Wechselspannung angelegt werden oder aber in Form einzelner Hochspannungspulse. Die Pulsfolgefrequenz bzw. die Wechselspannungsfrequenz kann zwischen etwa 10 Hz und etwa 30 kHz betragen. Die im Plasma umgesetzte Leistung kann mittels eines Messkondensators bestimmt werden, welcher die transferierten Ladungsträger eines Entladungszyklus integriert. Die so bestimmte Leistung kann zwischen etwa 0,5 Watt und etwa 5 Watt oder zwischen etwa 1 Watt und etwa 3 Watt betragen.

Der auf diese Weise erzeugte Plasmastrahl weist einen Durchmesser von etwa 0,15 mm bis etwa 0,5 mm auf. Beim Auftreffen auf dem Bauteil 2 weitet sich der Fußpunkt auf, sodass die zweite Teilfläche 22 größer sein kann als der Durchmesser des Plasmastrahles 4. Sofern die zweite Teilfläche 22 größer ist als der sich aus der Geometrie der Plasmaquelle ergebende Strahlfleck, kann durch Verschieben des Bauteils 2 bzw. der Gegenelektrode 51 mit dem darauf angeordneten Bauteil 2 eine größere zweite Teilfläche 22 durch sequenzielles Behandeln mit dem Plasma 4 bearbeitet werden. Der Abstand des Strahlaustritts von der zu behandlenden Oberfläche kann zwischen etwa 3 mm und etwa 8 mm betragen.

Anhand von Figur 6 wird die elipsometrisch bestimmte Schichtdickenabnahme der zweiten Teilfläche 22 durch die Plasmabehandlung erläutert. Dabei ist die Behandlungszeit mit dem anhand von Figur 5 erläuterten Plasmastrahl 4 auf der Abszisse aufgetragen sowie die elipsometrisch bestimmte Schichtdicke auf der Ordinate.

Wie Figur 6 zeigt, beträgt die Schichtdicke vor Einwirkung des Plasmas 110 nm. Nach einer Einwirkdauer von etwa 10 Sekunden verringert sich die Schichtdicke bereits auf etwa 75 nm. Nach 30 s beträgt die Schichtdicke etwa 57 nm. Bei sehr langer Behandlungsdauer von 300 Sekunden nimmt die Schichtdicke auf etwa 50 nm ab. Die Schichtdicke zeigt mit der Behandlungszeit einen asymptotischen Verlauf. Die in Figur 6 gezeigten Messwerte lassen vermuten, dass die Schichtdicke auch bei noch längerer Behandlungszeit nicht unter 50 nm fällt.

Damit zeigt Figur 6, dass mit der erfindungsgemäßen Plasmabehandlung der Beschichtung keine vollständige Entfernung der Beschichtung erreicht wird. Gleichwohl ändert sich die chemische Zusammensetzung und/oder die Bindungsverhältnisse der Konstituenten innerhalb der Beschichtung, wie nachfolgend noch näher erläutert werden wird. Damit einher geht eine Änderung des Benetzungsverhaltens. Die mit dem Plasma behandelte zweite Teilfläche ist nicht mehr wie ursprünglich hydrophob, sondern hydrophil, sodass die zweite Teilfläche 22 nach Einwirkung des Plasmas 4 dazu geeignet ist, mittels einer Klebeverbindung gefügt oder nochmals mit einem anderen Beschichtungsmaterial beschichtet zu werden.

Die in Figur 6 gezeigten Messwerte wurden anhand einer Beschichtung 3 ermittelt, welche durch Einbrennsilikonisierung erhalten wurde. Hierzu wird eine Emulsion aus Silikonöl und Wasser aufgetragen und nachfolgend durch eine Wärmebehandlung auf die Oberfläche des Bauteils 2 eingebrannt. Die nachfolgende Tabelle zeigt das Elementinventar der Beschichtung 3 vor der Einwirkung des Plasmas, nach 1 Sekunde, 10 Sekunden, 30 Sekunden, 60 Sekunden und nach 300 Sekunden. Alle Messwerte wurden mittels Photoelektronenspektroskopie erhalten. Dabei wird monochromatische Röntgenstrahlung auf die Oberfläche der Beschichtung 3 eingestrahlt und die kinetische Energie der Photoelektronen bestimmt. Aus der kinetischen Energie kann das jeweilige Element bestimmt werden, die Intensität der Photoelektronen gibt die relativen Anteile in der Beschichtung 3 an.

| **Element** | **0 s [Atom-%]** | **1 s [Atom-%]** | **10 s [Atom-%]** | **30 s [Atom-%]** | **60 s [Atom-%]** | **300 s [Atom-%]** |
|---|---|---|---|---|---|---|
| Sauerstoff (O) | 39,1 | 56,2 | 60,4 | 67 | 66,96 | 66,96 |
| Kohlenstoff (C) | 35,8 | 18,93 | 13,9 | 5,6 | 4,14 | 3,63 |
| Silizium (Si) | 25,1 | 24,85 | 25,7 | 27,4 | 28,68 | 29,28 |
| Rest | - - | - - | - - | - - | 0,27 | 0,13 |

Die Messungen wurden nach Einwirkung eines Atmosphärendruck-Plasmastrahls mit atmosphärischer Luft als Arbeitsgas erhalten, welcher beispielsweise mit der Vorrichtung nach Figur 5 erhältlich ist. Wie die vorstehend dargestellten Messwerte zeigen, nimmt der Kohlenstoffgehalt der Beschichtung mit zunehmender Einwirkdauer des Plasmas rasch ab. Dies ist darauf zurückzuführen, dass die im Silikon enthaltenen Methylgruppen abgespalten und durch den Gasstrom des Arbeitsgases des Plasmas 4 abtransportiert werden.

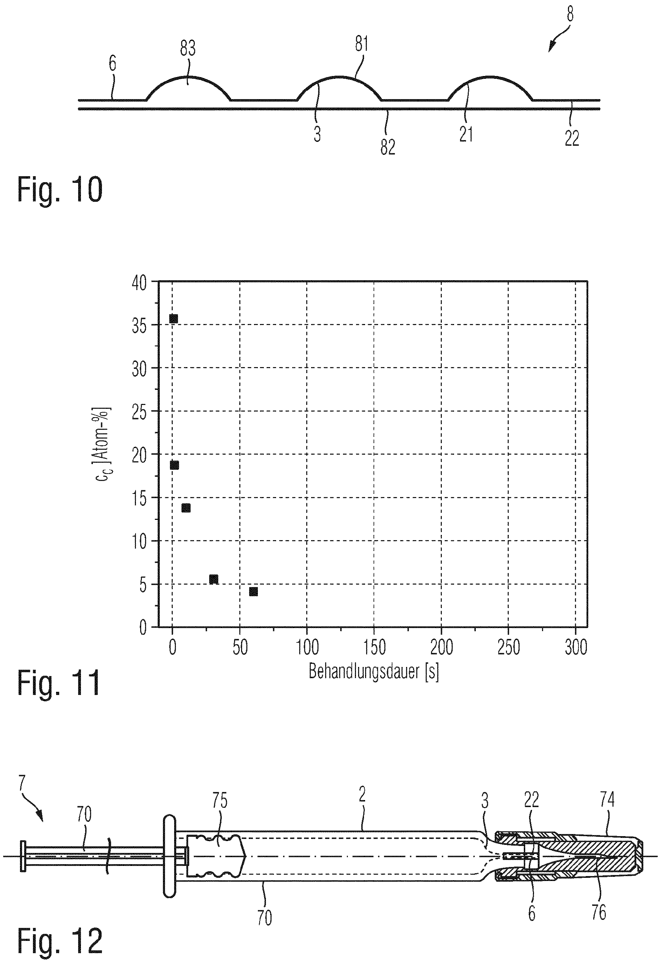

Figur 11 zeigt nochmals graphisch die Abnahme des Kohlenstoffgehaltes gegen die Behandlungsdauer. Dargestellt ist die Behandlungsdauer auf der Abszisse und der Kohlenstoff gehalt auf der Ordinate. Daraus ist ersichtlich, dass bereits sehr kurze Behandlungsdauern von weniger als einer Sekunde bis hin zu wenigen Sekunden einen signifikanten Effekt auf die Beschichtung ausüben. Solche Behandlungsdauern lassen sich insbesondere auch bei der Herstellung von Massenartikeln am Fließband einfach erreichen, ohne dass die Herstellung des Artikels dadurch wesentlich verzögert wird. Hierdurch kann die Haftfestigkeit einer nachfolgenden Lackierung und/oder Verklebung wesentlich verbessert werden, ohne dass gesundheitsgefährdende nasschemische Vefahren eingesetzt werden müssen.

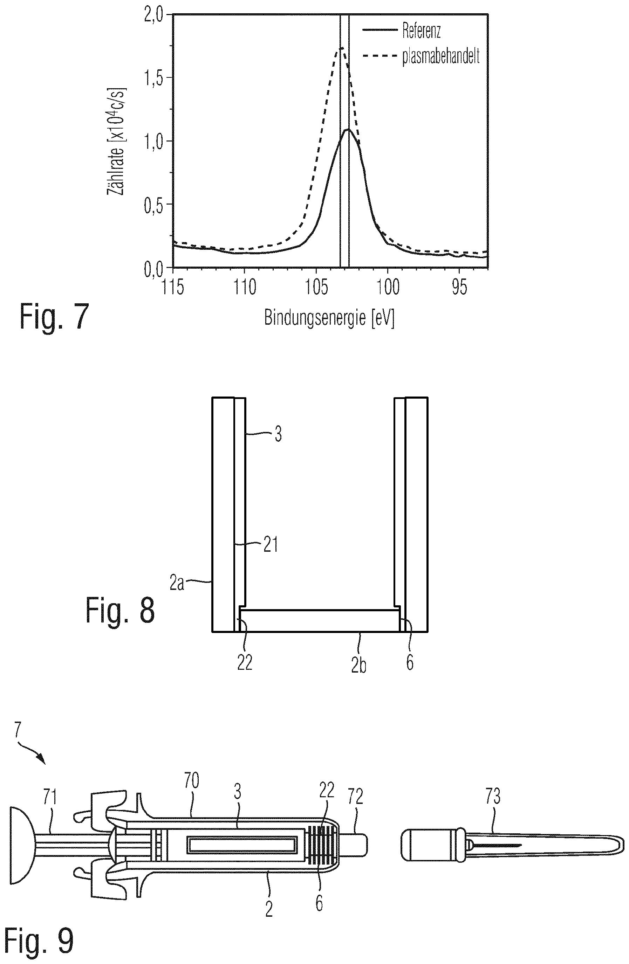

Figur 7 zeigt die energetische Lage des Silizium 2p-Peaks. Dabei ist die Bindungsenergie auf der Abszisse und die relative Häufigkeit bzw. Zählrate auf der Ordinate aufgetragen. Es ist ersichtlich, dass sich die Position des Silizium 2p-Niveaus durch die Plasmabehandlung von 102,6 eV auf 103,2 eV verschiebt. Der letztere Wert entspricht dem Literaturwert für Siliziumdioxid (SiO₂). Die polymere Silikonschicht verändert sich offensichtlich zu einer amorphen bzw. glasartigen Schicht.

Nachfolgend sollen mögliche Anwendungsfelder des erfindungsgemäßen Verfahrens näher erläutert werden. Dabei zeigt Figur 8 ein erstes Anwendungsbeispiel der Erfindung. Dargestellt ist in Figur 8 der Querschnitt durch einen in etwa zylindrischen Behälter mit einer zylindrischen Wandung 2a, welche beispielsweise aus einem Rohrabschnitt hergestellt werden kann. Die zylindrische Wandung 2a kann aus einem Glas oder Kunststoff hergestellt sein.

Auf die Innenseite der zylindrischen Wandung 2a wird eine Einbrennsilikonisierung als Beschichtung 3 aufgebracht, wie vorstehend beschrieben wurde. Hierzu wird eine Lösung bzw. eine Emulsion aus einem Silikonöl und einem Lösungsmittel aufgebracht und nachfolgend durch Behandlung im Wärmeofen bei etwa 150°C bis etwa 300°C eingebrannt. Die Beschichtung 3 weist hydrophobe Eigenschaften auf, sodass die Haftfestigkeit nachfolgender Klebeverbindungen beeinträchtigt ist.

An das erste Bauteil 2a soll ein zweites Bauteil 2b gefügt werden. Das zweite Bauteil 2b kann eine komplementär zur zylindrischen Wandung 2a geformte Bodenplatte sein, welche den so entstandenen Behälter nach unten fluiddicht abdichtet. Die Bodenplatte 2b soll mittels einer Klebeverbindung 6 befestigt und gedichtet werden.

Hierzu wird eine zweite Teilfläche 22 mit einem Atmosphärendruckplasma beaufschlagt, wie vorstehend beschrieben wurde. Dies führt einerseits zur Reduktion der Schichtdicke der Beschichtung 3. Gleichzeitig ändert sich die chemische Zusammensetzung und das Elementinventar der Beschichtung 3. Nach Abschluss der Plasmabehandlung ist die zweite Teilfläche 22 hydrophil. Dies sorgt für eine gute Haftfestigkeit der Klebeverbindung 6.

Auch wenn das zweite Bauteil 2b in Figur 8 ohne Beschichtung 3 dargestellt ist, so ist es selbstverständlich nicht ausgeschlossen, dass auch das Bauteil 2b vor dem Fügen mit einer Beschichtung versehen ist, welche ebenfalls durch Einbrennsilikonisieren aufgebracht werden kann. In diesem Fall kann auch die Klebefläche des Bauteils 2b nachfolgend einer Plasmabehandlung unterzogen werden, um die Haftfestigkeit zu verbessern oder um eine Klebeverbindung überhaupt erst zu ermöglichen.

Figur 9 zeigt eine Fertigspritze als zweites Ausführungsbeispiel der Erfindung. Die Fertigspritze 7 weist einen in etwa zylindrischen Spritzenkörper 70 auf. Der Körper 70 stellt ein erstes Bauteil 2 dar. Fertigspritzen 7 der dargestellten Art dienen als Verpackung des darin enthaltenen Medikamentes im Herstellwerk, sodass dieses unmittelbar gebrauchsfertig an den Arzt bzw. Patienten übergeben werden kann.

Zur Herstellung der in Figur 9 dargestellten Fertigspritze wird zunächst der Spritzenkörper 70 aus einem Glasrohr gefertigt. Hierzu wird das Glasrohr zugeschnitten, erwärmt und entsprechend der gewünschten Form umgeformt.

Im nächsten Verfahrensschritt wird zumindest die Innenseite mit einer Emulsion aus einem Lösungsmittel und schichtbildenden Substanzen besprüht und nachfolgend in einem Ofen bzw. Wärmeschrank behandelt. Dies führt dazu, dass ein Großteil des in der Emulsion vorhandenen Lösungsmittels verdampft. Gleichzeitig wird das als schichtbildende Substanz enthaltene Silikon kovalent an das Glas gebunden, sodass sich eine Beschichtung 3 auf der Innenseite ausbildet, welche Polysiloxan enthält oder daraus besteht. Durch die Wärmebehandlung wird vermieden, dass das Silikon beim späteren Befüllen, bei der Lagerung und beim Gebrauch der Fertigspritze 7 unerwünschterweise in das Medikament übergeht. Gleichzeitig erlaubt die Silikonisierung ein leichtes Gleiten des Kolbens 7, sodass die Handhabung der Fertigspritze 7 erleichtert ist.

In der dargestellten Ausführungsform wird an dem, dem Kolben 71 gegenüberliegenden Ende des Spritzenkörper 70 ein Konus 72 eingeklebt, welcher zur Aufnahme der Injektionsnadel 73 vorgesehen ist. In anderen Ausführungsformen der Erfindung kann die Injektionsnadel 73 auch unmittelbar in den Spritzenkörper 70 eingeklebt werden, sodass der Konus 72 auch entfallen kann.

Da die Beschichtung 3 auch die zur Aufnahme des Konus 72 vorgesehene zweite Teilfläche 22 bedeckt, ist die Haftfestigkeit einer Klebeverbindung 6 reduziert. Dies kann so weit gehen, dass der Konus 72 bereits beim Transport oder der Lagerung aus dem Spritzenkörper 70 herausfällt und der Inhalt der Fertigspritze 7 ausläuft.

Erfindungsgemäß wird daher vorgeschlagen, die zweite Teilfläche 22 in der vorstehend beschriebenen Weise mit einem Atmosphärendruckplasma zu behandeln und hierdurch die Beschichtung 3 zwar nicht vollständig zu entfernen, jedoch soweit zu inaktivieren, dass die Klebeverbindung 6 zuverlässig ausgefüllt werden kann. Durch die Änderung des Elementinventars und/oder der Bindungsverhältnisse der Konstituenten kann die hydrophobe Beschichtung 3 in der zweiten Teilfläche 22 hydrophil werden, um die Haftfestigkeit der Verklebung 6 zu verbessern.

Nach dem Einkleben des Konus 72 in den Spritzenkörper 70 wird die Fertigspritze 7 in an sich bekannter Weise zur Befüllung vorbereitet, d. h. gereinigt, sterilisiert und verpackt. Die auf diese Weise zur Befüllung vorbereitete Spritze wird dann an den Hersteller des Medikamentes zur Befüllung ausgeliefert.

Anhand der Figur 10 wird eine dritte Ausführungsform der vorliegenden Erfindung erläutert. Figur 10 zeigt den Querschnitt durch eine Blisterverpackung 8. Die Blisterverpackung enthält eine erste Folienlage 81, in welcher Kavitäten 83 ausgeformt sind. Die erste Folienlage 81 kann aus Aluminiumfolie oder Kunststoff geformt sein. Zumindest die Innenseite der Kavitäten 83 wird mit einer Einbrennsilikonisierung als Beschichtung 3 versehen, wie vorstehend erläutert wurde. Dies verhindert beim späteren Gebrauch der Blisterverpackung 8 das Anhaften des Packgutes und gleichzeitig den unerwünschten Übertritt des Silikonöls auf das Packgut.

Wenn die erste Folienlage 81 der Blisterverpackung vollständig silikonisiert wird, so verhindert dies eine zuverlässige Verklebung mit einer zweiten Folienlage 82, welche notwendig ist, um die Kavitäten 83 nach Einlegen des Packgutes zu verschließen. Daher werden zwischen den Kavitäten liegende zweite Teilflächen 22 in der beschriebenen Weise mit einem Plasma behandelt, um die Beschichtung 3 zu inaktivieren und von einem hydrophoben in einen hydrophilen Zustand zu überführen. Sodann können die Kavitäten 83 zuverlässig verschlossen werden, indem eine zweite, ebene Folienlage 82 auf die erste Folienlage 81 aufgeklebt wird.

Figur 12 zeigt ein Werkstück gemäß einer vierten Ausführungsform der Erfindung. Auch die vierte Ausführungsform ist eine Fertigspritze, deren prinzipielle Funktion bereits anhand der zweiten Ausführungsform erläutert wurde. Gleiche Bestandteile sind mit gleichen Bezugszeichen versehen, so dass sich die nachstehende Beschreibung auf die wesentlichen Unterschiede beschränkt.

Zur Herstellung der in Figur 12 dargestellten Fertigspritze (Staked In Needle Spritze) wird zunächst der Spritzenkörper 70 aus einem Glasrohr gefertigt. Hierzu wird das Glasrohr zugeschnitten, erwärmt und entsprechend der gewünschten Form umgeformt. Der Spritzenkörper 70 kann auch aus Kunststoff bestehen. Kunststoffspritzen werden durch bekannte Herstellverfahren, z.B. Spritzguss, gefertigt.

Im nächsten Verfahrensschritt wird zumindest die Innenseite des Spritzenkörpers 70 mit einer Emulsion aus einem Lösungsmittel und schichtbildenden Substanzen besprüht und nachfolgend in einem Ofen, z.B. einem Tunnelofen oder Wärmeschrank behandelt. Dies führt dazu, dass ein Großteil des in der Emulsion vorhandenen Lösungsmittels verdampft. Gleichzeitig wird das als schichtbildende Substanz enthaltene Silikon größtenteils kovalent an das Glas gebunden, sodass sich eine Beschichtung 3 auf der Innenseite ausbildet, welche Polysiloxan und/oder Polydimethylsiloxan enthält oder daraus besteht. Durch die Wärmebehandlung wird vermieden, dass das Silikon beim späteren Befüllen, bei der Lagerung und beim Gebrauch der Fertigspritze 7 unerwünscht in das Medikament übergeht. Gleichzeitig erlaubt die Silikonisierung ein leichtes Gleiten des Kolbenstopfens 75, sodass die Handhabung der Fertigspritze 7 bzw. eine Applikation des Medikamentes erleichtert oder überhaupt erst möglich wird.

In der dargestellten Ausführungsform wird an dem dem Kolbenstopfen 75 gegenüberliegenden Ende des Spritzenkörpers 70, eine Kanüle 76 eingeklebt.

Da die Beschichtung 3 auch die zur Aufnahme der Kanüle 76 vorgesehene zweite Teilfläche 22 bedeckt, ist die Haftfestigkeit einer Klebeverbindung 6 reduziert. Dies kann so weit gehen, dass die Kanüle 76 bereits beim Transport oder der Lagerung aus dem Spritzenkörper 70 herausfällt und der Inhalt der Fertigspritze 7 ausläuft.

Erfindungsgemäß wird daher vorgeschlagen, die zweite Teilfläche 22 in der vorstehend beschriebenen Weise mit einem Atmosphärendruckplasma zu behandeln und hierdurch die Beschichtung 3 zwar nicht vollständig zu entfernen, jedoch soweit zu inaktivieren, dass die Klebeverbindung 6, die in etwa genauso groß wie die Teilfläche 22 sein kann, zuverlässig ausgeführt werden kann. Durch die Änderung des Elementinventars und/oder der Bindungsverhältnisse der Konstituenten kann die hydrophobe Beschichtung 3 in der zweiten Teilfläche hydrophil werden, und so die Haftfestigkeit der Klebeverbindung 6 verbessern.

Nach dem Einkleben der Kanüle 76 in den Spritzenkörper 70 wird die Fertigspritze 7 in an sich bekannter Weise zur Befüllung vorbereitet, d.h. gereinigt, sterilisiert und verpackt. Hierbei wird auch das Nadelschutzteil 74 auf die Kanüle 76 aufgesetzt. Die auf diese Weise zur Befüllung vorbereitete Spritze wird dann an den Hersteller des Medikamentes zur Befüllung ausgeliefert.

Selbstverständlich ist die Erfindung nicht auf die dargestellten Ausführungsformen beschränkt. Die vorstehende Beschreibung ist daher nicht als beschränkend, sondern als erläuternd anzusehen. Die nachfolgenden Ansprüche sind so zu verstehen, dass ein genanntes Merkmal in zumindest einer Ausführungsform der Erfindung vorhanden ist. Dies schließt die Anwesenheit weiterer Merkmale nicht aus. Sofern die Ansprüche und die vorstehende Beschreibung "erste" und "zweite" Aus-führungsformen definieren, so dient diese Bezeichnung der Unterscheidung zweier gleichartiger Ausführungsformen, ohne eine Rangfolge festzulegen.

## Patentansprüche

1. Verfahren zur Beschichtung, bei welchem zumindest auf eine erste Teilfläche (21) einer Oberfläche eines Bauteils (2) zumindest eine Emulsion und/oder eine Lösung aufgebracht wird, welche zumindest eine schichtbildende Substanz enthält, und nachfolgend das Bauteil (2) wärmebehandelt wird, **dadurch gekennzeichnet, dass** zumindest eine zweite Teilfläche (22) der ersten Teilfläche (21) nachfolgend einem Plasma (4) ausgesetzt wird, wobei die zweite Teilfläche (22) auf der Innenfläche eines zylindrischen Hohlkörpers angeordnet ist und der Kohlenstoffgehalt der Beschichtung (3) auf weniger als 80% oder weniger als 75% oder weniger als 70% oder weniger als 60% des Ausgangswertes vor der Plasmabehandlung abnimmt, wobei
zur Erzeugung des Plasmas eine Wirbelströmung des Arbeitsgases zum Einsatz kommt.

2. Verfahren nach Anspruch 1, **dadurch gekennzeichnet, dass** die Schichtdicke der Beschichtung (3) zumindest in der zweiten Teilfläche (22) vor der Einwirkung des Plasmas zwischen 20 nm und 100 nm oder zwischen 30 nm und 70 nm beträgt und durch die Plasmabehandlung in der zweiten Teilfläche (22) um mehr als 20% oder mehr als etwa 25 % oder mehr als 30 % abnimmt und/oder
dass nach der Plasmabehandlung in der zweiten Teilfläche (22) eine Schichtdicke von mehr als 70% oder mehr als 60 % oder mehr als 50 % verbleibt.

3. Verfahren nach Anspruch 1 oder 2, **dadurch gekennzeichnet, dass** die Emulsion und/oder die Lösung zumindest ein Silikonöl und optional Wasser enthält oder daraus besteht und/oder
dass die Beschichtung (3) zumindest Kohlenstoff und Sauerstoff und Wasserstoff und Silicium enthält und/oder dass die Beschichtung (3) zumindest ein Poly(organo)siloxan enthält.

4. Verfahren nach einem der Ansprüche 1 bis 3, **dadurch gekennzeichnet, dass** die zweite Teilfläche (22) vor der Plasmabehandlung hydrophob und nach der Plasmabehandlung hydrophil ist.

5. Verfahren nach einem der Ansprüche 1 bis 4, **dadurch gekennzeichnet, dass** dass das Plasma (4) ein Aktivgas enthält oder daraus besteht und
dass das Aktivgas optional Sauerstoff oder synthetische Luft oder atmosphärische Luft enthält oder daraus besteht.

6. Verfahren nach einem der Ansprüche 1 bis 4, **dadurch gekennzeichnet, dass** das Plasma (4) für 0,4 bis 5 Sekunden oder für 0,5 bis 4 Sekunden oder für 0,5 bis 1,5 Sekunden oder für 5 bis 60 Sekunden einwirkt und/oder dass ein Atmosphärendruckplasma zum Einsatz kommt, welches mit einer dielektrisch behinderten Entladung erzeugt wird und/oder
dass das Plasma (4) ein Inertgas enthält oder daraus besteht und
dass das Inertgas optional ein Edelgas, insbesondere Argon, enthält oder daraus besteht und/oder dass das Plasma als Plasma-strahl oder Plasmajet ausgebildet ist, welcher zumindest auf die zweite Teilfläche einwirkt.

7. Verfahren zur Herstellung eines Werkstücks (1) mit zumindest einem ersten Bauteil (2a, 70, 81) und zumindest einem zweiten Bauteil (2b, 72, 76, 82), welche miteinander gefügt werden, wobei zumindest ein Bauteil (2a, 70, 81) mit einem Verfahren nach einem der Ansprüche 1 bis 6 beschichtet wird.

8. Verfahren nach Anspruch 7, **dadurch gekennzeichnet, dass** die zweite Teilfläche (22) der ersten Teilfläche (21) zumindest die Fügestelle (6) umfasst.

9. Verfahren nach einem der Ansprüche 7 oder 8, **dadurch gekennzeichnet, dass** das Fügen unter Verwendung eines Klebstoffes erfolgt, welcher insbesondere ausgewählt ist aus einem Acrylat und/oder einem Polyurethan und/oder einem Epoxidharz und/oder einem Cyanacrylat.

10. Werkstück (1) mit zumindest einem ersten Bauteil (2a, 70, 81) und zumindest einem zweiten Bauteil (2b, 72, 76, 82), welche durch Kleben miteinander gefügt sind, wobei die Fügestelle (6) zumindest eine zweite Teilfläche (22) einer ersten Teilfläche (21) der Oberfläche des ersten Bauteils (2a, 70, 81) umfasst, **dadurch gekennzeichnet, dass** zumindest auf die erste Teilfläche (21) der Oberfläche des ersten Bauteils (2a, 70, 81) zumindest eine Beschichtung (3) aufgebracht ist, wobei zumindest die zweite Teilfläche (22) der ersten Teilfläche (21) nachfolgend einem Plasma ausgesetzt wurde, wodurch der Kohlenstoffgehalt der Beschichtung in der zweiten Teilfläche auf weniger als 80 % des Ausgangswertes vor der Plasmabehandlung reduziert ist und wobei die zweite Teilfläche (22) auf der Innenfläche eines zylindrischen Hohlkörpers angeordnet ist.

11. Werkstück nach Anspruch 10, **dadurch gekennzeichnet, dass** die Schichtdicke der Beschichtung (3) zumindest in der zweiten Teilfläche (22) vor der Einwirkung des Plasmas zwischen 20 nm und 100 nm oder zwischen 30 nm und 70 nm beträgt und durch die Plasmabehandlung in der zweiten Teilfläche (22) um mehr als 20% oder mehr als 25 % oder mehr als 30 % abnimmt und/oder
dass nach der Plasmabehandlung in der zweiten Teilfläche (22) eine Schichtdicke von mehr als 70% oder mehr als 60 % oder mehr als 50 % verbleibt.

12. Werkstück nach Anspruch 10 oder 11, **dadurch gekennzeichnet, dass** die Beschichtung (3) zumindest Kohlenstoff und Sauerstoff und Wasserstoff und Silicium enthält und/oder dass die Beschichtung (3) zumindest ein Poly(organo)siloxan enthält und/oder
dass der Kohlenstoffgehalt der Beschichtung (3) auf weniger als 75% oder weniger als 70% oder weniger als 60% des Ausgangswertes vor der Plasmabehandlung reduziert ist.

13. Werkstück nach einem der Ansprüche 10 bis 12, **dadurch gekennzeichnet, dass** das Fügen unter Verwendung eines Klebstoffes erfolgt, welcher insbesondere ausgewählt ist aus einem Acrylat und/oder einem Polyurethan und/oder einem Epoxidharz und/oder einem Cyanacrylat.

14. Werkstück nach einem der Ansprüche 10 bis 13, **dadurch gekennzeichnet, dass** das Werkstück (1) ausgewählt ist aus einer Fertigspritze (7) und/oder einer Verpackung (8) und/oder einer Maschinenkomponente.

## Claims

1. Method for coating, in which at least a first partial area (21) of a surface of a component (2) is coated with at least one emulsion and/or a solution containing at least one coating-forming substance, and the component (2) is subsequently heat-treated, **characterized in that** at least a second partial area (22) of the first partial area (21) is subsequently exposed to a plasma (4), wherein the second partial area (22) is arranged on the inner surface of a cylindrical hollow body and the carbon content of the coating (3) decreases to less than 80% or less than 75% or less than 70% or less than 60% of the initial value prior to the plasma treatment, wherein a vortex flow of the working gas is used to generate the plasma.

2. Method according to claim 1, **characterized in that** the layer thickness of the coating (3) is between 20 nm and 100 nm or between 30 nm and 70 nm at least in the second partial area (22) prior to the exposure of the plasma and decreases by more than 20% or more than about 25% or more than 30% in the second partial area (22) as a result of the plasma treatment and/or
**in that** a layer thickness of more than 70% or more than 60% or more than 50% remains in the second partial area (22) after the plasma treatment.

3. Method according to claim 1 or 2, **characterized in that** the emulsion and/or the solution comprises or consists of at least one silicone oil and optionally water and/or
**in that** the coating (3) contains at least carbon and oxygen and hydrogen and silicon and/or
**in that** the coating (3) contains at least one poly(organo)siloxane.

4. Method according to any one of claims 1 to 3, **characterized in that** the second partial area (22) is hydrophobic prior to the plasma treatment and is hydrophilic after the plasma treatment.

5. Method according to any one of claims 1 to 4, **characterized in that** the plasma (4) contains or consists of an active gas and
**in that** the active gas optionally contains or consists of oxygen or synthetic air or atmospheric air.

6. Method according to any one of claims 1 to 4, **characterized in that** the plasma (4) acts for 0.4 to 5 seconds or for 0.5 to 4 seconds or for 0.5 to 1.5 seconds or for 5 to 60 seconds and/or
**in that** an atmospheric pressure plasma is used, which is generated by means of a dielectrically impeded discharge and/or
**in that** the plasma (4) contains or consists of an inert gas and
**in that** the inert gas optionally contains or consists of a noble gas, in particular argon, and/or
**in that** the plasma is formed as a plasma beam or plasma jet which acts at least on the second partial area.

7. Method for producing a workpiece (1) having at least a first component (2a, 70, 81) and at least a second component (2b, 72, 76, 82), which are joined together, wherein at least one component (2a, 70, 81) is coated by means of a method according to any one of claims 1 to 6.

8. Method according to claim 7, **characterized in that** the second partial area (22) of the first partial area (21) comprises at least the joint (6).

9. Method according to any one of claims 7 or 8, **characterized in that** the joining is carried out using an adhesive which is in particular selected from an acrylate and/or a polyurethane and/or an epoxy resin and/or a cyanoacrylate.

10. Workpiece (1) having at least a first component (2a, 70, 81) and at least a second component (2b, 72, 76, 82), which are joined together by adhesion, the joint (6) comprising at least a second partial area (22) of a first partial area (21) of the surface of the first component (2a, 70, 81), **characterized in that** at least the first partial area (21) of the surface of the first component (2a, 70, 81) is coated with at least one coating (3), wherein at least the second partial area (22) of the first partial area (21) has subsequently been exposed to a plasma, as a result of which the carbon content of the coating in the second partial area is reduced to less than 80% of the initial value prior to the plasma treatment and wherein the second partial area (22) is arranged on the inner surface of a cylindrical hollow body.

11. Workpiece according to claim 10, **characterized in that** the layer thickness of the coating (3) is between 20 nm and 100 nm or between 30 nm and 70 nm at least in the second partial surface (22) prior to the exposure of the plasma and decreases by more than 20% or more than 25% or more than 30% as a result of the plasma treatment in the second partial area (22) and/or
**in that** a layer thickness of more than 70% or more than 60% or more than 50% remains in the second partial area (22) after the plasma treatment.

12. Workpiece according to claim 10 or 11, **characterized in that** the coating (3) contains at least carbon and oxygen and hydrogen and silicon and/or
**in that** the coating (3) contains at least one poly(organo)siloxane and/or
**in that** the carbon content of the coating (3) is reduced to less than 75% or less than 70% or less than 60% of the initial value prior to the plasma treatment.

13. Workpiece according to any one of claims 10 to 12, **characterized in that** the joining is carried out using an adhesive which is in particular selected from an acrylate and/or a polyurethane and/or an epoxy resin and/or a cyanoacrylate.

14. Workpiece according to any one of claims 10 to 13, **characterized in that** the workpiece (1) is selected from a pre-filled syringe (7) and/or a packaging (8) and/or a machine component.

## Revendications

1. Procédé de revêtement, dans lequel au moins une émulsion et/ou une solution contentant au moins une substance formant une couche est appliquée au moins sur une première surface partielle (21) d'une superficie d'un composant (2), et ensuite le composant (2) est traité thermiquement, **caractérisé en ce qu'**au moins une deuxième surface partielle (22) de la première surface partielle (21) est ensuite exposée à un plasma (4), la deuxième surface partielle (22) étant disposée sur la face intérieure d'un corps creux cylindrique et la teneur en carbone du revêtement (3) diminuant jusqu'à moins de 80 % ou moins de 75 % ou moins de 70 % ou moins de 60 % de la valeur initiale avant le traitement au plasma, et
un écoulement tourbillonnaire du gaz de travail est utilisé pour générer le plasma.

2. Procédé selon la revendication 1,
**caractérisé en ce que** l'épaisseur de couche du revêtement (3) est comprise entre 20 nm et 100 nm ou entre 30 nm et 70 nm, au moins dans la deuxième surface partielle (22) avant l'action du plasma, et diminue de plus de 20 % ou de plus d'environ 25 % ou de plus de 30 % par le traitement au plasma dans la deuxième surface partielle (22), et/ou
**en ce que**, après le traitement au plasma, une épaisseur de couche de plus de 70 % ou de plus de 60 % ou de plus de 50 % reste dans la deuxième surface partielle (22).

3. Procédé selon la revendication 1 ou 2,
**caractérisé en ce que** l'émulsion et/ou la solution contient ou est constituée d'au moins une huile de silicone et éventuellement d'eau, et/ou **en ce que** le revêtement (3) contient au moins du carbone et de l'oxygène et de l'hydrogène et du silicium, et/ou
**en ce que** le revêtement (3) contient au moins un poly(organo)siloxane.

4. Procédé selon l'une des revendications 1 à 3,
**caractérisé en ce que** la deuxième surface partielle (22) est hydrophobe avant le traitement au plasma et est hydrophile après le traitement au plasma.

5. Procédé selon l'une des revendications 1 à 4,
**caractérisé en ce que** le plasma (4) contient ou est constitué d'un gaz actif, et
**en ce que** le gaz actif contient ou est constitué optionnellement d'oxygène ou d'air synthétique ou d'air atmosphérique.

6. Procédé selon l'une des revendications 1 à 4,
**caractérisé en ce que** le plasma (4) agit pendant 0,4 à 5 secondes, ou pendant 0,5 à 4 secondes, ou pendant 0,5 à 1,5 secondes, ou pendant 5 à 60 secondes, et/ou
**en ce que** l'on utilise un plasma à pression atmosphérique, qui est produit par une décharge à barrière diélectrique, et/ou
**en ce que** le plasma (4) contient ou est constitué d'un gaz inerte, et
**en ce que** le gaz inerte contient ou est constitué optionnellement d'un gaz rare, en particulier d'argon, et/ou
**en ce que** le plasma est conçu comme un faisceau de plasma ou comme un jet de plasma, qui agit au moins sur la deuxième surface partielle.

7. Procédé de fabrication d'une pièce (1) avec au moins un premier composant (2a, 70, 81) et au moins un deuxième composant (2b, 72, 76, 82), qui sont assemblés l'un à l'autre, au moins un composant (2a, 70, 81) étant revêtu par un procédé selon l'une des revendications 1 à 6.

8. Procédé selon la revendication 7,
**caractérisé en ce que** la deuxième surface partielle (22) de la première surface partielle (21) présente au moins l'emplacement d'assemblage (6).

9. Procédé selon l'une des revendications 7 ou 8,
**caractérisé en ce que** l'assemblage est réalisé en utilisant une colle choisie en particulier parmi un acrylate et/ou un polyuréthane et/ou une résine époxy et/ou un cyanoacrylate.

10. Pièce (1) avec au moins un premier composant (2a, 70, 81) et au moins un deuxième composant (2b, 72, 76, 82), qui sont assemblés l'un à l'autre par collage, au moins une deuxième surface partielle (22) d'une première surface partielle (21) de la superficie du premier composant (2a, 70, 81) présentant l'emplacement d'assemblage (6),
**caractérisée en ce qu'**au moins un revêtement (3) est appliqué sur la première surface partielle (21) de la superficie du premier composant (2a, 70, 81), au moins la deuxième surface partielle (22) de la première surface partielle (21) ayant été ensuite exposée à un plasma, ce qui réduit la teneur en carbone du revêtement dans la deuxième surface partielle à moins de 80 % de la valeur initiale avant le traitement au plasma, et la deuxième surface partielle (22) est disposée sur la face intérieure d'un corps creux cylindrique.

11. Pièce selon la revendication 10,
**caractérisée en ce que** l'épaisseur de couche du revêtement (3) est comprise entre 20 nm et 100 nm ou entre 30 nm et 70 nm, au moins dans la deuxième surface partielle (22) avant l'action du plasma, et diminue de plus de 20 % ou de plus de 25 % ou de plus de 30 % par le traitement au plasma dans la deuxième surface partielle (22), et/ou
**en ce que**, après le traitement au plasma, une épaisseur de couche de plus de 70 % ou de plus de 60 % ou de plus de 50 % reste dans la deuxième surface partielle (22).

12. Pièce selon la revendication 10 ou 11,
**caractérisée en ce que** le revêtement (3) contient au moins du carbone et de l'oxygène et de l'hydrogène et du silicium, et/ou
**en ce que** le revêtement (3) contient au moins un poly(organo)siloxane, et/ou **en ce que** la teneur en carbone du revêtement (3) est réduite jusqu'à moins de 75 % ou moins de 70 % ou moins de 60 % de la valeur initiale avant le traitement au plasma.

13. Pièce selon l'une des revendications 10 à 12,
**caractérisée en ce que** l'assemblage est réalisé en utilisant une colle choisie en particulier parmi un acrylate et/ou un polyuréthane et/ou une résine époxy et/ou un cyanoacrylate.

14. Pièce selon l'une des revendications 10 à 13,
**caractérisée en ce que** la pièce (1) est choisie parmi une seringue prête à l'emploi (7) et/ou un emballage (8) et/ou un composant de machine.
